Europäisches Patentamt

European Patent Office

Office européen des brevets

(19)

(11) Veröffentlichungsnummer: **0 205 077**
**A2**

# (12) EUROPÄISCHE PATENTANMELDUNG

(21) Anmeldenummer: **86107525.7**

(22) Anmeldetag: **03.06.86**

(51) Int. Cl.⁴: **A 61 K 31/185**

(30) Priorität: **11.06.85 DE 3520846**

(43) Veröffentlichungstag der Anmeldung:
**17.12.86 Patentblatt 86/51**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Stünkel, Klaus Georg, Dr.**
**Am Eckbusch 55**
**D-5600 Wuppertal 1(DE)**

(72) Erfinder: **Grützmann, Rudi, Dr.**
**Helsinkistrasse 20**
**D-5650 Solingen(DE)**

(54) Suramin-Natrium zur Verwendung als Immunstimulans.

(57) Die Erfindung betrifft Suramin-Natrium zur Verwendung als Immunstimulans, immunstimulierende Arzneimittel, enthaltend Suramin-Natrium, sowie Verfahren zur Herstellung solcher Arzneimittel.

EP 0 205 077 A2

Croydon Printing Company Ltd.

BAYER AKTIENGESELLSCHAFT    5090 Leverkusen, Bayerwerk
Konzernverwaltung RP
Patentabteilung    Ad/Kü-c

Suramin-Natrium zur Verwendung als Immunstimulans

Die Erfindung betrifft Suramin-Natrium zur Verwendung als
Immunstimulans, immunstimulierende Arzneimittel, enthaltend Suramin-Natrium sowie Verfahren zur Herstellung
solcher Arzneimittel.

Suramin-Natrium entspricht der chemischen Verbindung 8,8'-
[Carbonylbis[imino-3,1-phenylencarbonylimino(4-methyl-3,1-
phenylen)carbonylimino]] bis -1,3,5-naphthalintrisulfon-
saures hexa-Natriumsalz gemäß The Merck Index, 10th
Edition, Nr. 8890, die bislang mit Erfolg gegen Trypanosomen und Filarien eingesetzt wurde.

Suramin-Natrium und seine Herstellung gehören zum bekannten Stand der Technik. Als Literatur, die sich mit
Suramin-Natrium befaßt, seien beispielsweise genannt:
Fourneau et al., Compt. Rend, 178, 675 (1924);
GB-PS 224 849; Heymann, Angewandte Chemie 37 585 (1924);
Olenick, Antibiotics Vol. 3; J.W. Corcoran, F.E. Hahn,
Eds. (Springer Verlag, New York, 1975) pp 699-703.

Le A 23 892 - Ausland

Es wurde nun gefunden, daß das bekannte Suramin-Natrium immunstimulierende Wirkung aufweist.

Gegenstand der Erfindung ist das 8,8'-[Carbonylbis[imino-3,1-phenylencarbonylimino(4-methyl-3,1-phenylen)carbonyl-imino]] bis -1,3,5-naphthalin-trisulfonsaures hexa-Natriumsalz (= Suramin-Natrium entsprechend Merck Index, 10. Auflage, Nr. 8890) zur Verwendung als Immunstimulans bei der therapeutischen Behandlung des menschlichen oder tierischen Körpers.

Weiterer Gegenstand der Erfindung sind Arzneimittel, enthaltend Suramin-Natrium als Immunstimulans, zur therapeutischen Behandlung des menschlichen oder tierischen Körpers.

Weitere Gegenstände der Erfindung sind die Verwendung von Suramin-Natrium als Immunstimulans bzw. zur Behebung von Immunschwächen des menschlichen oder tierischen Körpers, die Verwendung von Suramin-Natrium zur Herstellung von immunstimulierenden Arzneimitteln sowie Verfahren zur Herstellung von immunstimulierenden Arzneimitteln, in denen man Suramin-Natrium gemäß der Definition in Anspruch 1 mit inerten, nicht-toxischen, pharmazeutisch geeigneten Trägerstoffen mischt.

Suramin-Natrium weist eine ausgeprägte abwehrsteigernde Wirkung auf. Es zeigte sich, daß das Suramin-Natrium die Antikörpersynthese des Immunsystems Antigen-spezifisch steigert und die T-Lymphocytenproliferation erhöht. Diese Ergebnisse wurden anhand der nachfolgenden Versuchsanord-nung erhalten.

Le A 23 892

Steigerung der primären humoralen Immunität _in vitro_
gegen Schaferythrozyten (SE)

Es ist experimentell möglich, die Entwicklung einer humoralen Immunantwort mit heterologen roten Blutzellen durch
primäre Immunisation von Maus-Milzzellen in Suspensionskulturen _in vitro_ einzuleiten (R.I. Mishell und
R.W Dutton, J. Exp. Med. 126, 423 (1967)).

Hierzu werden Balb/c Maus-Milzzellen für 5 Tage in Gegenwart von Antigen (SE) und der Testsubstanz kultiviert. Die
Zellen werden geerntet, gewaschen und zusammen mit dem
Antigen und Komplement in semisolidem Agar ausplattiert
und für 2 Stunden bei 37°C inkubiert (N.K. Jerne.
A.A. Nordin und C. Henry, "Cell bound Antibodies", eds,
Amos and Koprowski, Wistar Inst. Press, Philadelphia, USA,
p 109(1963)). Die Antigen-Sensibilisierung von Maus-
Lymphozyten in der Primärkultur resultiert in der Synthese
und Freisetzung von Antikörpern. Die spezifischen, sezernierten Antikörper binden sich an das SE-Antigen und
lysieren diese Zellen durch die Gegenwart des Komplements
(Plaque-Bildung). Suramin-Natrium ist in der Lage,
dosisabhängig im Bereich 0,1-30 µg/ml die Zahl der Antikörper-bildenden Zellen zu steigern (Tabelle 1).

Le A 23 892

**Tabelle 1** Einfluß von Suramin-Natrium auf die Antikörper-synthese in vitro

| Substanz | Antikörper-sezernierende Zellen/Kultur in Abhängigkeit von der Dosis (µg/ml) | | | | | | |
|---|---|---|---|---|---|---|---|
| | 0 | 0,1 | 0,3 | 1 | 3 | 10 | 30 |
| Suramin-Natrium | 60 | 195 | 960 | 890 | 1125 | 1270 | 2155 |

Steigerung der primären humoralen Immunität in vivo

NMRI-Mäuse wurden mit einer suboptimalen Antigendosis (1 µg/Tier) subcutan (s.c.) immunisiert. Bei suboptimaler Antigenstimulation wird nur eine geringe Zahl von Lymphozyten der Tiere zur Antikörpersynthese angeregt. Die zusätzliche Behandlung der Tiere mit der erfindungsgemäßen Verbindung ist in der Lage, bei einer einmaligen und auch bei wiederholter Applikation von 3-30 mg/kg subcutan den Antikörpertiter im Serum der Tiere signifikant zu steigern. Die Antikörperbestimmung erfolgt durch indirekte Hämagglutination am Tag 10. Der Effekt der Behandlung wird durch den geometrischen Mittelwert der $\log_2$-Titer ausgedrückt.

Le A 23 892

Steigerung der unspezifischen, Mitogen-induzierten Stimulation von Maus-Milzlymphozyten

Separierte Milzzellen naiver, unbehandelter Balb/c-Mäuse wurden mit dem T-Lymphozyten-Mitogen PHA (10 µg/ml) und dem B-Lymphozyten-Mitogen LPS (50 µg/ml) unspezifisch stimuliert. Nach 48 Stunden Kulturdauer wurden die Kulturen mit $^3$H-Thymidin versetzt und nach weiteren 16 Stunden geerntet. Die Inkorporation des radioaktiven Thymidins in die neu synthetisierte DNA gilt als Maß für die erfolgte Lymphozytenprofileration. Die Ergebnisse zeigen eine deutliche Proliferationszunahme im Dosisbereich von 1-100 µg/ml (Tab. 2).

Tabelle 2 Einfluß von Suramin-Natrium auf die unspezifische, mitogene Stimulation von Maus-Milzlymphozyten

|  | Dosis (µg/ml) | | | | |
|  | 1 | 3 | 10 | 30 | 100 |
|  | $^3$H-Thymidin-Einbau (% der Kontrolle) | | | | |
| Ø | 70 | 90 | 70 | 120 | 70 |
| PHA | 140 | 140 | 140 | 200 | 230 |
| LPS | 130 | 140 | 140 | 220 | 160 |

Le A 23 892

Folgende Patientengruppen können beispielsweise mit Erfolg mit Suramin-Natrium behandelt werden:

Patienten, die an Infektions- (Virus, Bakterien, Pilze) oder Alters-bedingten, Tumor- induzierten oder durch Cytostatika hervorgerufenen Immunschwächen leiden. Außerdem kommen postoperative und posttraumatische immunologische Abwehrschäden vor. Die Behandlung von Patienten mit solchen erworbenen Immundefekten ist nicht befriedigend gelöst. Alle diese Patienten benötigen Medikationen, die die körpereigene Abwehr unterstützen bzw. normalisieren. Dieses läßt sich überraschenderweise mit Suramin-Natrium erreichen.

Zur vorliegenden Erfindung gehören pharmazeutische Zubereitungen, die neben nicht toxischen, inerten pharmazeutisch geeigneten Trägerstoffen Suramin-Natrium enthalten oder die aus Suramin-Natrium bestehen sowie Verfahren zur Herstellung dieser Zubereitungen.

Le A 23 892

Zur vorliegenden Erfindung gehören auch pharmazeutische Zubereitungen des Suramin-Natriums in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können, z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können Suramin-Natrium neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B.

Le A 23 892

- 8 -

Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenden Überzügen und Hüllen versehen sein und auch so zusammengesetzt sein, daß sie das Suramin-Natrium nur oder bevorzugt in einem bestimmten Teil des Intestinaltraktes, gegebenenfalls verzögert abgeben, wobei als Einbettungsmassen, z.B. Polymersubstanzen und Wachse verwendet werden können.

Suramin-Natrium kann gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyethylentglykole, Fette, z.B. Kakaofett und höhere Ester (z.B. $C_{14}$-Alkohol mit $C_{16}$-Fettsäure) oder Gemische dieser Stoffe.

Le A 23 892

Puder und Sprays können neben Suramin-Natrium die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben Suramin-Natrium die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Poylethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben Suramin-Natrium die üblichen Trägerstoffe wie flüssige Verdünnungsmittel, z.B. Wasser, Ethylalkohol, Propylenglykol, Suspensiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Le A 23 892

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Suramin-Natrium soll in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer Suramin-Natrium auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffs mit dem oder den Trägerstoffen.

Zur vorliegenden Erfindung gehört auch die Verwendung von Suramin-Natrium sowie von pharmazeutischen Zubereitungen, die den Wirkstoff enthalten, in der Human- und Veterinärmedizin zur Verhütung, Besserung und/oder Heilung der oben angeführten Erkrankungen.

Der Wirkstoff oder die pharmazeutischen Zubereitungen können lokal, oral, parenteral, intraperitoneal und/oder rectal, vorzugsweise parenteral, insbesondere intravenös appliziert werden.

Le A 23 892

Im allgemeinen hat es sich sowohl in der Human- as auch in der Veterinärmedizin als vorteilhaft erwiesen, Suramin-Natrium in Gesamtmengen von etwa 1,0 bis 300, vorzugsweise 3,0 bis 30 mg/kg, insbesondere 2,5 bis 25 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

Le A 23 892

Patentansprüche

1.  8,8'-[Carbonylbis[imino-3,1-phenylencarbonylimino
    (4-methyl-3,1-phenylen)carbonylimino]] bis -1,3,5-
    naphthalin-trisulfonsaures hexa-Natriumsalz (= Suramin-Natrium) zur Verwendung als Immunstimulans bei
    der therapeutischen Behandlung des menschlichen
    oder tierischen Körpers.

2.  Arzneimittel, enthaltend Suramin-Natrium nach
    Anspruch 1 als Immunstimulans, zur therapeutischen
    Behandlung des menschlichen oder tierischen Körpers.

3.  Verwendung von Suramin-Natrium nach Anspruch 1 als
    Immunstimulans bzw. zur Behebung von Immunschwächen
    des menschlichen oder tierischen Körpers.

4.  Verwendung von Suramin-Natrium nach Anspruch 1 zur
    Herstellung von immunstimulierenden Arzneimitteln.

5.  Verfahren zur Herstellung von immunstimulierenden
    Arzneimitteln, dadurch gekennzeichnet, daß man
    Suramin-Natrium gemäß der Definition in Anspruch 1
    mit inerten, nicht-toxischen, pharmazeutisch
    geeigneten Trägerstoffen mischt.

Le A 23 892